Europäisches Patentamt

European Patent Office

Office européen des brevets

(11) Publication number: **0 177 000**
A1

# (12) EUROPEAN PATENT APPLICATION

(21) Application number: **85112360.4**

(51) Int. Cl.⁴: **A 61 K 9/52**

(22) Date of filing: **30.09.85**

(30) Priority: **02.10.84 IT 2295484**

(43) Date of publication of application:
**09.04.86 Bulletin 86/15**

(84) Designated Contracting States:
**AT BE CH DE FR GB IT LI LU NL SE**

(71) Applicant: EURAND ITALIA S.p.A.
Via Privata Pasteur 1
I-20092 Cinisello Balsamo, Milano(IT)

(72) Inventor: Calanchi, Massimo
Via Calatafimi, 12
I-20052 Monza, Milano(IT)

(72) Inventor: Gentilini, Leonardo
Via Alessandro Paoli, 4
I-20124 Milano(IT)

(74) Representative: Riccardi, Sergio
Riccardi & Co. Via Macedonio Melloni, 32
I-20129 Milano(IT)

(54) Process to obtain a sustained release formulation of water soluble components.

(57) The dissolution of water soluble substances, more particularly of drugs, can be delayed by a process consisting of: mixing and/or granulating the water soluble substance with macromolecules that swell in water and dissolve only slowly in an excess of water; coating such granules with a water insoluble polymeric membrane that allows the diffusion by osmosis of the encapsulated substances once dissolved into water, or into the gastro-intestinal juices in the case of drugs.

EP 0 177 000 A1

-1-

"Process to obtain a sustained release formulation of water soluble components"

In the pharmaceutical fields, but even in other fields, it is often useful and advantageous to slacken the dissolution of the active substances in order to extend the absorption time in the gastro-intestinal tract and consequently to extend the therapeutic action, as well as to avoid or reduce side effects.

One of the most frequently used methods for this purpose is to coat the drug particles with a polymeric film using physical or chemical-physical methods.

Among the first methods it may for instance be cited the application of a membrane by spraying a solution of it on the particles of the product to be coated and then evaporating the solvent.

Among the second methods, one of the most common systems is the microencapsulation by coacervation or phase separation.

When the substances to be coated have a very high solubility in water or in aqueous solution such as the gastric and enteric juices, it is practically impossible to succeed in obtaining that the dissolution of the active substance after coating occurs in some hours instead of few minutes or seconds. In such cases in order to attain this purpose it is generally used to apply membrane amounts in excess of 50% or to apply fat and/or wax increasing membrane imperviousness. However, in this way the process becomes technically complex and expensive, has poor reproducibility and often the finished product has also an insufficient bioavailability. Acceptable results were in few cases obtained by coating substances in the form of big crystals (such as with KCl), but in practice this is very rare because the raw materials and more particularly the organic ones, are found on the market mainly in the form of powder, and also because one would in any case be strictly bound to the crystal size and the range of distribution of their particle size.

Thus granulation of the substances before coating was employed, but also in this case the results are often unsatisfactory, because it is anyway necessary to use high percentages of membrane and/or to apply also fat or wax, so as to be again faced with the above mentioned problems.

The present invention therefore relates to a technically and economically advantageous method for extending up to 12 hours or even more, the release of substances which are highly soluble in water and aqueous solutions starting from a conventional pharmaceutical form such as granules.

The process of the invention consists in the steps of:

(A) granulating the soluble substance with polymers of high molecular weight having the characteristic of swelling in water, forming a viscous gel-like layer, and then of dissolving slowly in presence of an excess of water; and

(B) subsequently coating the granule so obtained, by applying around it a polymeric membrane with a method based on microencapsulation by phase separation or even by simply spraying a polymer solution, said membrane being water insoluble but allowing release of the active substance by diffusion, so that the gastro-intestinal juices pass through the membrane, dissolve the drug and the solution goes out through said

- 3 -

0177000

membrane.

It is known to use hydrocolloids, that is macromolecules which are hydrated in presence of water and are gelling and then dissolve in an excess of liquid, to obtain sustained release products.

For instance USP 4 167 558 and 4 126 672 to Steth et al relate to the preparation of tablets or granules containing hydrocolloids, which are floating in the stomach and slowly release the active substance. In this case floating in the stomach is emphasized and the applications relate to drugs to be absorbed therein.

Also USP 4 389 393 to Schor et al relates to the preparation of retard drugs with hydrocolloids but it is limited to preparation of tablets.

USP 4 235 870 to Leslie discloses the preparation of sustained release granules to be used for capsules or tablets, but in this case the object is attained by mixing in well defined proportions hydroxyalkylcellulose which must be previously hydrated, and long chain alifatic alcohols.

GB-P-2 053 681 to Kawata et al deals with a sustained release pharmaceutical composition containing the drug in the amorphous form and polymers such as polymers of ethylene oxide, carboxypolymethylene (Carbopol), hydroxypropylmethylcellulose and others, but in this disclosure, like in the other above cited patents, there is no subsequent microencapsulation of the granules and therefore they cannot be used to slacken dissolution of highly water soluble drugs.

USP 3 415 758 to Powell et al, USP 3 155 590 to Miller et al and GB-P 931 148 to Upjohn Company generally relate to microencapsulation processes by phase separation. They are applicable to water soluble or insoluble substances, but there is no particular reference to the composition or the characteristics of the products to be coated, excepting the obvious fact that they are compatible with the solvents and the

0177000

excipients used in the process.

Finally FR-P 2 392 667 to Imperial Chemical Industries Ltd. discloses a method for preparing a sustained release drug (propanolol), but the method illustrated to render granules like spheres and the subsequent coating of said granules with ethylcellulose in a coating pan, does not mention use of hydrocolloids in the granule, but merely cites hydroxypropylmethylcellulose as possibly used together with ethylcellulose as a coating agent of the granule containing the drug.

Detailed description of the process

The substance, of which it is desired to slacken dissolution in water or aqueous solutions, which may be either a drug or not, is placed in a suitable mixer for wet granules, such as a discontinuous Leodige mixer with vortex centrifugal operation, or a Turbo-Sphere mixer made by TS Pharma or a high speed granulator mixer made by T.K. Fielder Ltd. or merely a four-way blendor or a planetary mixer or finally a fluidized bed granulator.

Then a solution of hydrocolloids is added, i.e. of ,macromolecules having the characteristic of swelling in water by forming a gel and of dissolving in an excess of water or aqueous solution in order to wet the mass homogeneously.

The obtained granules are used just as they are or they are sieved or passed through a machine reducing the particle size such as an oscillating granulator.

The hydrocolloid solution may be in an organic solvent or preferably in an aqueous solvent and the hydrocolloids may be used alone or in mixture.

The hydrocolloids may also be mixed, wholly or partially, in dry form with the drug just as it is or with the drug absorbed on an inert excipient or mixed with it. In such a case the subsequent granulation may be dry by compaction for instance with a compacting machine Alexander Werke, and subsequent breaking of the compacted product, for instance with an oscillating granulator or preferably with a wet operation. In the wet granulation it is possible to use the solution of any binder generally used for this purpose or preferably the solution of a hydrocolloid. In the latter case it is possible to use a solution in organic or preferably aqueous solvent, of the same hydrocolloid already mixed with the drug, also in dry form or another hydrocolloid or a mixture of two or more hydrocolloids.

The so prepared drug granules anyway dissolve in a short time and therefore in order to slacken the dissolution to 12 or more hours, it is necessary to coat them with a polymeric membrane which is insoluble in the gastro-intestinal juices but allows diffusion. Obviously one should use a sufficiently resilient membrane, which does not break when the granules swell.

The gastro-intestinal juices penetrate through the pores of the membrane enveloping the granules containing the drug, the hydrocolloid and possibly an inert vehicle, swell the hydrocolloid (gelling) and dissolve the drug. The solution of those components diffuses from the interior to the exterior of the microcapsules and finally is absorbed. The presence of the hydrocolloid gel reduces greatly the velocity with which juices contact the drug and consequently extends the drug dissolution time.

On the other hand the hydrocolloid dissolves in an excess of juices so that all the drug may be released from the granule and once in solution, may diffuse through the membrane.

The release of the substances from the microcapsules (although drugs are generally mentioned herein, it is obvious that the same technique is applicable to any product soluble in water or aqueous solvents of which it is desired to slacken dissolution) is therefore slackened by two different processes, namely diffusion of the dissolved substances through the membrane of the microcapsules, which must remain intact, and swelling with slow dissolution of the hydrocolloids added in the granulation stage.

It is conceivable that swelling of hydrocolloid macromolecules may partially clog the pores of the membrane of the microcapsules so as to slacken diffusion of liquids both to the interior and the exterior of the microcapsules and this could be an explanation of the phenomenon.

Granulation of substances with macromolecules swelling in water and gelling and the subsequent microencapsulation of the granules causes both processes to work at the same time so as to slacken dissolution to 12 or more hours even in case of substances which are highly soluble in water or aqueous solutions.

Coating or microencapsulation of the granules may be effected by applying techniques already known in the art. As illustrative but non limiting examples only, presently preferred techniques are described hereinafter:

(1) Microencapsulation by phase separation. In this case coating of the granules occurs by phase separation of the membrane forming polymer following for instance one of the methods disclosed in the above cited USP 3 415 758 and 3 155 590 and GB-P 931 148. The process generally consists in the steps of:

(a) dissolving the membrane forming polymer in a suitable solvent or microencapsulation vehicle;

(b) suspending in it the granules prepared as above described, keeping them stirred with a suitable stirrer;

(c) causing phase separation of the membrane forming polymer with one of the known methods or a combination thereof; the most common methods are the following: temperature reduction, addition of membrane non-solvent, addition of other polymer more soluble in the vehicle than said membrane forming polymer, pH variation; in this stage the membrane forming polymer is deposited around the granules coating them

- 7 -                                          0177000

with a continuous film;

(d) possibly hardening the membrane;

(e) separating microcapsules from vehicle, for instance by filtration or centrifugation, and drying said microcapsules.

(2) Microencapsulation by spray in fluidized bed. In the fluidized bed a heated air flow is passing, keeping the granules agitated while a membrane solution is being sprayed on them. The continuous motion of the granules causes the spray to wet the entire granule surface on which, once the solvent is evaporated, the membrane is deposited. Membrane concentration in the solution, amount of sprayed solution per time unity, flow and temperature of air circulating in the apparatus, are regulated so as to obtain a homogeneous membrane deposition.

As non limiting example of drugs to which the above described process may be applied the following are cited: methoclopramide HCl, phenylephrine HCl, D-pseudoe-phedrine HCl, dextromethorphane HBr, phenylpropanolamine HCl, aminophillin, cimetidine, isosorbide-5-mononitrate, codein phosphate, soberal, lithium sulphate, lithium carbonate, clorphemiramine maleate, diphenhydramine HCl, oxeladine citrate, procainamide HCl, diclofenac Na, thiopronine, buflomedil, sulfenazone, flecainide acetate, potassium guaiacosulphonate, diethylpropione.

Obviously the process may be applied also to non pharmaceutical highly water soluble substances, of which it is desired to slacken the dissolution in water or aqueous solutions.

Finally, even in case that the product, either drug or not, does not show a high water solubility, this process may anyway offer technical and economical advantages when compared to similar processes, as granulation with hydrocolloids allows to use a considerably lower membrane in the coating stage.

The polymers used for granulation and/or directly mixed in powder form are straight chain polymers of high molecular weight that in water firstly swell giving rise to formation of a viscous gel, which in excess of water or aqueous solutions slowly decreases in viscosity and then dissolves completely. These polymers are also called hydrocolloids and among them the following are cited as a non limiting example: methylcellulose, hydroxyethylcellulose, hydroxypropylcellulose, hydroxypropylmethylcellulose, sodium carboxymethylcellulose, carboxypolymethylene (Carbopol 934 of Goodrich Chemical Co.), agar-agar, carragenine, alginates, arabic gum, guar gum, carob gum, gum tragacanth, karaja gum, gelatine, casein, zein, polyvinyl alcohols, polyvinylpyrrolidone and its derivatives, modified starch.

The inert excipients possibly mixed or on which the drug is absorbed before granulation, preferably are water insoluble. As a non limiting illustrative example the following are cited: levilite, bentonite, microcrystalline cellulose (Avical made by FMC), methylcellulose (Elcema by Degussa), silicium dioxide (Syloid by Davison, Aerosil by Degussa, Cab-O-Sil by Cabot), attapulgite.

The polymer preferably used to coat granules is ethylcellulose. Other polymers and/or plasticizers may be mixed therewith. As a non limiting illustrative example other polymers are now cited, which can be used in lieu of ethylcellulose, such as: polyacrylates and polymethacrylates, polyvinylidene (Saran), shellac, nitrocellulose, polyvinylchloride, polyethylene, poliamides, silicones.

The following practical examples of application of the process should be construed merely as illustrative of the invention, without limiting at all by them the scope and the objects of said invention.

EXAMPLE 1

(A) Granulation

In a beaker a solution is prepared in ethyl alcohol, containing 3.5% ethylcellulose and 31.0% methoclopramide.

This solution is added in a laboratory kneader where previously were mixed levilite and hydrocolloids, hydroxypropylmethylcellulose (Methocel E 50 and K 4 M of Dow Chemical) and hydroxypropylcellulose (Klucel LF of Hercules) in a ratio 5.5 : 1.

The material, possibly after being passed through an oscillating granulator, is dried in a fluidized bed and then sieved.

Fraction comprised between 850 and 210 microns is used for subsequent coating by coacervation or phase separation.

(B) Microencapsulation

In a beaker containing cyclohexane, ethylcellulose as a membrane and polyethylene as phase separator are added under continuous agitation. Heat up to 75°C and keep stirring until dissolution of ethylcellulose and polyethylene is complete.

The previously prepared granulate is then added (ratio granulate/ethylcellulose is 3:1). Cool slowly up to 25°C.

The obtained microcapsules are washed with hexane and separated by filtration, then dried on fluidized bed.

The microcapsules analyzed in vitro with the apparatus described in USP XX, page 959, gave the following results:

Titre : 179.0 mg/g (in base)

Release : 1 hour 10.2% - 4 hours 50.9% - 8 hours 75.8%

Microcapsules of methoclopramide prepared in the same way, but with a granule not containing hydrocolloids, when analyzed in vitro under the same conditions, gave the following results:

titre : 183.0 mg/g (in base)

release : 1 hour 35.2% - 4 hours 72.5%

## EXAMPLE 2

(A) Granulation

In a beaker a solution is prepared in ethyl alcohol, containing 3.5% ethylcellulose and 31.0% methoclopramide.

This solution is added in a laboratory kneader where levilite and hydrocolloid, hydroxypropylmethylcellulose (Methocel 15M of Dow Chemical) were previously mixed in a ratio 5.5 : 1. Then the procedure of Example 1(A) is repeated.

(B) Microencapsulation

The procedure of Example 1(B) is repeated, excepting the ratio granulate/ethylcellulose that in this case is 5:1.

The microcapsules analyzed in vitro with the apparatus described in USP XX, page 959 gave the following results:

titre: 206.3 mg/g (in base)

release : 1 hour 26.1% - 4 hours 77.8% - 8 hours 95.3 %

Microcapsules of methoclopramide prepared in the same way, but with a granulate not containing hydrocolloid, when analyzed in vitro under the same conditions, gave the following results:

titre : 235.0 mg/g (in base)

release : 1 hour 56.0% - 4 hours 97.0%

## EXAMPLE 3

(A) Granulation

In a beaker a solution in distilled water is prepared, containing 3.5% hydrocolloid, hydroxypropylmethylcellulose (Methocel E 50 of Dow Chemical) and 31.0% methoclopramide.

- 11 -

This solution is added in a laboratory kneader in which levilite was previously loaded (ratio levilite/hydrocolloid 20:1). The procedure of the Example 1(A) is then repeated.

(B) Microencapsulation

The procedure described in Example 1(B) is repeated excepting the ratio granulate/ethylcellulose which in this case is 6.5:1.

The microcapsules analyzed in vitro with the apparatus described in USP XX page 959, gave the following results:

titre : 225.0 mg/g (in base)

release : 1 hour 15.6% - 4 hours 56.1% - 8 hours 75.4%

The microencapsulation test was repeated twice with higher amounts of granulate with the following results:

titre : 212.7 mg/g (in base)

release : 1 hour 18.5% - 4 hours 61.0% - 8 hours 79.8%

titre : 214.2 mg/g (in base)

release : 1 hour 20.7% - 4 hours 63.2% - 8 hours 77.5%

Microcapsules of methoclopramide prepared in the same way, but with a granulate not containing a hydrocolloid, when analyzed in vitro under the same conditions, gave the following results:

titre : 219.0 mg/g (in base)

release : 1 hour 63.0% - 2 hours 83.2%

EXAMPLE 4

(A) Granulation

In a beaker a solution in distilled water is prepared, containing 3.0% hydrocolloids, hydroxypropylmethylcellulose (Methocel E 50 and K4 M of Dow Chemical) and hydroxypropylmethylcellulose (Klucel LF of Hercules) and 26.0% methoclopramide.

This solution is added in a laboratory kneader in which levilite was previously loaded (ratio levilite/hydrocolloid 21:1). The procedure of Example 1(A) is then repeated.

(B) Microencapsulation

The procedure described in Example 2(B) in then repeated.

The microcapsules analyzed in vitro with the apparatus described in USP XX, page 959 gave the following results:

titre : 191.6 mg/g (in base)

release : 1 hour 7.0% - 4 hours 37.5% - 8 hours 57.3%

EXAMPLE 4 bis

The same granulate of the preceding Example (4A) was coated by applying a membrane of ethylcellulose in a fluidized bed instead of coacervation. Ethylcellulose was dissolved at 5% in acetone/isopropanol (ratio 1:1) and sprayed under the following conditions:

air pressure : 2.5 atm

spraying speed: 30 rpm

inlet air temperature : 50°C

outlet air temperature : 35-40°C

In this example the ratio granulate/ethylcellulose is 5:1. The microcapsules analyzed in vitro with the apparatus described in USP XX, page 959, gave the following results:

titre : 185.2 mg/g (in base)

release : 1 hour 15% - 4 hours 40.3% - 8 hours 58.2%

## EXAMPLE 5

(A) Granulation

In a beaker a solution in distilled water is prepared, containing 6.0% hydroxypropylmethylcellulose (Methocel E 15 of Dow Chemical) and 29.0% methoclopramide.

This solution is added in a laboratory kneader in which levilite had previously been loaded (ratio levilite/hydrocolloid 10:1). The procedure of Example 1(A) is then repeated.

(B) Microencapsulation

The procedure described in Example 2(B) is repeated. The microcapsules analyzed in vitro with the apparatus described in USP XX, page 959 gave the following results:

titre : 216.0 mg/g (in base)

release : 1 hour 18.0% – 4 hours 58.0% – 8 hours 77.0%

## EXAMPLE 6

(A) Granulation

In a beaker a solution in distilled water is prepared, containing 5% hydroxypropylmethylcellulose (Methocel E 50 of Dow Chemical).

This solution is added in a laboratory kneader in which aminophillin had been previously loaded (ratio aminophyllin/hydrocolloid 24 : 1). The procedure of Example 1(A) is then repeated.

(B) Microencapsulation

The procedure described in Example 1(B) is followed, excepting the ratio granulate/ethylcellulose that in this case is 4:1. Microcapsules analyzed in vitro with the apparatus Diffutest (Eurand) gave the following results:

titre : 765.0 mg/g (in base)

release : 1 hour 21.0% – 4 hours 55.0% – 8 hours 68.0%

EXAMPLE 7

(A) Granulation

A solution in ethyl alcohol is prepared, containing 10% polyvinylpirrolidone (Kollidon of BASF).

This solution is added in a laboratory kneader in which a mixture of phenylpropanolamine chlorohydrate and gum tragacanth in a ratio 2:1 was previously loaded.

The procedure of Example 1(A) is then repeated.

(B) Microencapsulation

The procedure of Example 1(B) is followed, excepting the ratio granulate/ethylcellulose which in this case is 6:1.

Microcapsules analyzed in vitro with the apparatus described in USP XX, page 959 gave the following results:

titre : 520.0 mg/g (in base)

release : 1 hour 23.2% - 4 hours 54.4% - 8 hours 83.7%

Having thus completely described the process according to the present invention, with the aid of the examples of application given above, it is to be understood that many variations, modifications, additions and/or substitutions may be resorted to said process, without departing however from its spirit and object and without falling outside the scope of protection, as it results defined in the appended claims.

- 1 -

0177000

CLAIMS

1) Process for slackening dissolution of substances soluble in water or in aqueous solutions, characterized by the fact of comprising the following steps: (a) mixing said substances with polymers swelling in water and then dissolving in an excess of water; (b) granulating the mixture so obtained; (c) coating the granules with a membrane which is insoluble in water and aqueous solutions, but allows diffusion of the coated substances.

2) Process for slackening dissolution of substances soluble in water or in aqueous solutions, characterized by the fact of comprising the following steps: (a) granulating said substances with solutions or suspensions of polymers swelling in water and then dissolving in a water excess; (b) coating the granules with a membrane which is insoluble in water and aqueous solutions but allows diffusion of the coated substance.

3) Process according to Claim 1, characterized by the fact that an inert substances is added to the mixture before granulation.

4) Process according to Claim 1 or 2, characterized by the fact that the substances where slackening of dissolution is desired, are highly water soluble drugs.

5) Process according to Claim 1 or 2, characterized by the fact that the substance where slackening of dissolution is desired, is methoclopramide HCl.

6) Process according to Claim 1 or 2, characterized by the fact that the polymers swelling in water are chosen from the group comprising methylcellulose, hydroxypropylmethylcellulose, hydroxypropylcellulose, sodium carboxymethylcellulose, carboxypolymethylene, alginates, gum tragacanth, polyvinyl alcohols, polyvinylpirrolidone.

7) Process according to Claim 1, characterized by the fact that granulation is effected by adding solutions of polymers swelling in water and then dissolving in an excess of water.

8) Process according to Claim 1 or 2, characterized by the fact that coating of the granules is effected by coacervation and phase separation.

9) Process according to Claim 1 or 2, characterized by the fact that coating of the granules is effected by spraying the membrane in a fluidized bed.

10) Process according to Claim 1 or 2, characterized by the fact that the applied membrane is ethylcellulose.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 077 956 (TANABE SEIYAKU CO., LTD.) * Page 5, line 17 - page 6, line 9; page 6, line 17 - page 7, line 1; page 7, line 18 - page 8, line 4 * | 1-4,6, 8,10 | A 61 K 9/52 |
| Y | | 5 | |
| X | FR-A-2 148 045 (THE NATIONAL CASH REGISTER CO.) * Page 1, lines 22-27; page 3, lines 5-27; page 4, lines 3-5; page 4, lines 12-16; page 4, line 39 - page 5, line 9; page 5, lines 12-26 * | 1,2,4, 6-8,10 | |
| X | US-A-3 362 880 (SAMPSON F. JEFFRIES) * Column 1, lines 29-38; column 1, lines 57-62; column 2, lines 40-43 * | 1-4,6, 7,10 | TECHNICAL FIELDS SEARCHED (Int. Cl.4) A 61 K 9/52 A 61 K 9/54 A 61 K 9/62 A 61 K 9/66 |
| X | US-A-3 400 185 (KENNETH J. KOHNLE et al.) * Column 2, lines 20-30; column 5, lines 16-31, 39-48; claims 5,8 * | 1,2,4, 6,10 | |
| Y | WO-A-8 201 468 (LARUELLE CLAUDE) * Claim 1 * | 5 | |

---  -/-

The present search report has been drawn up for all claims

| Place of search THE HAGUE | Date of completion of the search 03-12-1985 | Examiner TZSCHOPPE,D.A. |
|---|---|---|

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | US-A-4 153 677 (PHILIP M. JOHN)<br><br>* Column 3, line 55 - column 4, line 4; column 4, line 59 - column 5, line 4 *<br><br>--- | 1-3,6,<br>8,10 | |
| A | GB-A-1 085 739 (PARKE, DAVIS AND CO.)<br>* Page 2, line 117 - page 3, line 1; page 3, lines 66-79; claims 1,3 *<br><br>--- | 1,2,6,<br>10 | |
| D,A | GB-A- 931 148 (UPJOHN CO.)<br><br>* Page 4, lines 117-128; page 4, lines 65-76; claims 2,11,14,15 *<br><br>----- | 1,2,8,<br>10 | |

TECHNICAL FIELDS
SEARCHED (Int. Cl.4)

The present search report has been drawn up for all claims

| Place of search<br>THE HAGUE | Date of completion of the search<br>03-12-1985 | Examiner<br>TZSCHOPPE,D.A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82